# FASCICULE DE BREVET EUROPEEN

(11) **EP 1 311 670 B1**
(45) Date de publication et mention de la délivrance du brevet: **21.02.2007**
(21) Numéro de dépôt: 01965350.0
(22) Date de dépôt: 24.08.2001
(51) Int. Cl.: C12N 15/10, C12N 15/11

(54) **PROCEDE DE MUTAGENESE DIRIGEE MASSIVE**
VERFAHREN ZUR ORTSPEZIFISCHEN MASSENMUTAGENESE
METHOD FOR MASSIVE DIRECTED MUTAGENESIS

(30) Priorité: 25.08.2000 FR 0010962
(43) Date de publication de la demande: 21.05.2003
(73) Titulaire: Biomethodes, 91000 Evry (FR)
(72) Inventeur: DELCOURT, Marc, F-94800 Villejuif (FR); BLESA, Stéphane, F-77680 Roissy-en-Brie (FR)
(74) Mandataire: Casalonga, Axel
(86) Numéro de dépôt international: PCT/FR2001/002666
(87) Numéro de publication internationale: WO 2002/016606

(56) Documents cités:
- US-A- 5 798 208
- XIAO, G. ET AL.: "Construction and screening of a multi-point site-specific mutant library of subtilisin E with a set of oligonucleotides" SCIENCE IN CHINA, SERIES C: LIFE SCIENCES, vol. 40, no. 4, 1997, pages 337-344, XP000993443

## Description

La présente invention concerne le domaine de la biologie moléculaire et plus particulièrement celui de la mutagenèse. Elle a pour objet un procédé de mutagenèse dirigée à haut débit, c'est-à-dire la constitution de nombreux mutants dirigés en un temps et un nombre d'étapes réduits. Ce procédé sera donc qualifié de « mutagenèse massive ».

La mutagenèse est une technique visant à modifier artificiellement la séquence nucléotidique d'un fragment d'ADN dans le but de modifier l'activité biologique qui en découle. La mutagenèse a pris cette dernière décennie une place importante dans de nombreux travaux de biologie moléculaire.

Le terme dé mutagenèse peut être associé à trois modifications distinctes d'un fragment d'ADN :
- la délétion, qui consiste à éliminer des nucléotides du fragment d'ADN d'intérêt,
- l'insertion, qui consiste à en rajouter, et
- la substitution, qui consiste à remplacer une
ou plusieurs bases par un même nombre de bases de nature différente.

Les techniques de mutagenèse peuvent être séparées en deux grands groupes : la mutagenèse aléatoire d'une part, et la mutagenèse dirigée d'autre part.

La mutagenèse aléatoire vise à introduire des substitutions de nature et de position aléatoires dans un fragment d'ADN. Historiquement, la mutagenèse aléatoire était réalisée au moyen de procédés chimiques altérant la structure de l'ADN. Plus récemment, l'amplification d'une molécule en utilisant une polymérase dans des conditions particulières a souvent remplacé les procédés chimiques. Ces conditions particulières sont caractérisées en ce qu'elles altèrent les capacités dé l'enzyme à répliquer fidèlement l'ADN. Celle-ci introduit au fil des cycles des mutations, c'est-à-dire des différences par rapport à la séquence initiale. À la fin de la réaction, un grand nombre de copies de la molécule initiale sont obtenues, chacune de ces molécules comportant des mutations différentes. Ces molécules sont présentes sous forme d'une banque, c'est-à-dire d'un mélange de molécules de nature différente (différant par la nature et la position de leurs mutations).

La mutagenèse dirigée vise à introduire une ou quelques mutations (substitutions, mais aussi délétions ou insertions) de nature et de position connues dans un fragment d'ADN. Un oligonucléotide est utilisé pour introduire cette mutation. Cet oligonucléotide est classiquement constitué d'une vingtaine de bases. La séquence de cet oligonucléotide est homologue en tout point à la séquence ciblée sur le fragment d'ADN à l'exception d'une ou de quelques positions localisées dans sa partie médiane.

Cet oligonucléotide est ensuite utilisé pour amorcer une réaction de réplication (ou d'amplification, c'est-à-dire de multiples réplications) en utilisant le fragment d'ADN comme matrice. La séquence nouvellement synthétisée contient la modification recherchée.

Les premières techniques de mutagenèse dirigée étaient basées sur l'amplification du seul fragment d'ADN d'intérêt (sous la forme d'un fragment d'ADN linéaire), qui devait ensuite être introduit dans un plasmide. Ces techniques étaient fastidieuses et devaient être adaptées à chaque système d'étude.

Plus récemment, l'oligonucléotide mutant a été utilisé pour répliquer directement le plasmide contenant le fragment d'ADN d'intérêt. Le nombre de manipulations à réaliser est ainsi minimisé.

La réalisation pratique de la mutagenèse dirigée est cependant loin d'être simple. Se pose notamment le problème de l'isolement des molécules ayant incorporé la mutation par rapport aux molécules ne l'ayant pas incorporée. La seule réplication d'un fragment d'ADN circulaire (correspondant au cas classique d'un gène cloné dans un plasmide) au moyen d'un oligonucléotide mutant ne permet pas, en l'absence de système de sélection, d'observer un taux de mutagenèse détectable. L'ADN synthétisé *in vitro* pouvant être distingué de l'ADN synthétisé dans des bactéries sur le critère de son contenu en bases méthylées, un système de criblage basé sur ce critère a été mis au point et généralisé. Il s'agit d'utiliser l'enzyme DpnI, spécifique de sites présents sur l'ADN méthylé mais pas sur l'ADN non méthylé (Lacks et al., 1980, Methods in Enzymology, 65 :138). Les molécules n'ayant pas subi de réplication *in vitro* sont ainsi éliminées. Mais même en utilisant ce système de criblage de mutants, l'efficacité de la réaction de mutagenèse reste faible et voisine de 5% seulement de molécules mutantes.

Ce faible taux de mutants est notamment dû au fait qu'à l'issue de la réaction de mutagenèse dirigée, les molécules circulaires sont introduites dans des bactéries contenant un système de réparation de l'ADN qui élimine une grande partie des mutations si celles-ci ne sont portées que par l'un des brins d'ADN.

De nombreux systèmes ont été proposés pour tenter d'améliorer l'efficacité de ces techniques de mutagenèse. Ces techniques nécessitent le plus souvent un second oligonucléotide permettant d'améliorer la fréquence des molécules mutantes avant criblage (Brevet EP 96942905 ; Brevet WO 9935281). D'autres systèmes utilisent également un second oligonucléotide permettant un système de criblage particulier et parfois plus efficace (Brevet EP 0938552, Catalogue Clontech 2000, page 45). Enfin d'autres systèmes utilisent des souches bactériennes particulières, ces systèmes ayant pour objet de minimiser la déperdition de rendement due à l'activité réparatrice des bactéries (Brevet US 4,873,192 ; Brevet EP 0938552).

Enfin, la plupart des techniques existantes permettent d'intégrer simultanément plusieurs oligonucléotides dans une séquence d'ADN. En règle générale, jusqu'à trois oligonucléotides ont pu être introduits simultanément en différents lieux du fragment à muter (Brevet WO 9935281 ; Brevet EP 0938552). Un article fait même référence à l'obtention de molécules ayant simultanément intégré jusqu'à sept oligonucléotides en une seule étape (Perlak et al., 1990, Nucleic Acid Research, 18 :7457).

La notion de banque n'est pas, dans la technique selon l'art antérieur, adaptée pour caractériser les produits obtenus à l'issue d'une réaction de mutagenèse dirigée. En effet, dans la grande majorité des cas, un seul produit est obtenu à l'issue de la réaction, contenant une seule mutation. Le brevet US5,798,208 décrit une méthode de mutagenèse dans laquelle un acide aminé choisi à l'avance est introduit à toutes les positions possibles d'une région sélectionnée d'une protéine afin de produire une banque de mutants de cette protéine, le but étant d'évaluer le rôle d'un acide aminé spécifique dans la structure ou la fonction d'une protéine donnée. Dans les cas où plusieurs mutations sont introduites simultanément au moyen de plusieurs oligonucléotides (Brevet WO 9935281 ; Perlak et al., 1990, Nucleic Acid Research, 18 :7457), les seuls produits recherchés sont ceux ayant incorporé la totalité des mutations, et la technique tend à être optimisée dans l'objectif de maximiser la fréquence de ces produits. Les produits n'ayant incorporé qu'une petite fraction des mutations sont minimisés et sont dans ces rapports considérés comme des produits secondaires.

La mutagenèse peut avoir pour but le gain ou la perte d'une activité.

Le gain d'une activité, ou plus fréquemment sa simple amélioration, est particulièrement intéressante dans les domaines de l'enzymologie ou dé l'association ligand/récepteur. Ainsi, pouvoir disposer d'une enzyme d'activité améliorée peut permettre de réduire les coûts de procédés industriels utilisant ces enzymes. De même, l'affinité de la liaison entre un ligand et son récepteur peut être améliorée grâce à quelques mutations localisées au niveau du site de reconnaissance de l'un par l'autre.

La recherche de ces améliorations d'activité est souvent désignée de façon générique par « évolution moléculaire ». Il s'agit de simuler l'évolution lors de réactions *in vitro,* en introduisant des mutations dans un fragment d'ADN et en sélectionnant ceux ayant des activités améliorées. Plusieurs tours de mutagenèse/sélection miment ainsi l'évolution d'une molécule en présence d'une pression sélective.

Le type de mutagenèse utilisé le plus fréquemment dans ce contexte est la mutagenèse aléatoire. En effet, aucun élément ne permettant généralement de définir à priori la nature et la position des changements susceptibles d'apporter une amélioration de l'activité étudiée, il est nécessaire dans ce contexte de produire un grand nombre de molécules ayant chacune des mutations de position et de nature différentes, afin de maximiser les chances que parmi elles se trouve une molécule correspondant à une activité améliorée.

La perte de l'activité biologique associée à un fragment d'ADN ayant été soumis à mutagenèse apporte des informations particulières sur les acides aminés supportant son activité. Ainsi, si la modification d'un acide aminé entraîne la perte de l'activité biologique, il est probable que cet acide aminé intervienne dans la constitution du site actif supportant cette activité biologique. Ces résultats doivent cependant être considérés avec beaucoup de nuances : il est par exemple possible que cet acide aminé n'intervienne pas directement dans le site actif de l'activité biologique, mais qu'il prenne part à des activités annexes, comme l'adressage intracellulaire de la protéine par exemple. D'autre part, il est possible que la modification introduite déstabilise l'ensemble de la protéine, l'effet de la substitution introduite étant alors indirect et non direct. Pour ces deux raisons, il est important de savoir reconnaître sur un gène codant pour une protéine les motifs supportant les activités d'adressage, de localisation membranaire, de liaison de cofacteurs... D'autre part, il est essentiel que les modifications introduites induisent le moins de déstabilisation possible de la protéine. Le plus souvent, ce sont de petits acides aminés hydrophobes, l'Alanine ou la Valine, qui sont introduits en substitution des acides aminés d'origine. Ces petits acides aminés sont connus pour conserver la majorité des structures secondaires protéiques (Hélice α ou feuillet β), et donc pour minimiser les déstabilisations globales des protéines.

Les travaux menés dans ce domaine impliquent la création d'un grand nombre de mutants ponctuels portant chacun une substitution différente d'un acide aminé de la protéine par une alanine. Ces travaux nécessitent une somme de travail très importante, chaque mutant devant être réalisé indépendamment.

Il existe des exceptions aux cas généraux présentés ci-dessus. La mutagenèse dirigée a été utilisée dans le contexte de la recherche d'un gain d'activité. Dans le cas où la région du site actif est bien connue, on peut en effet espérer que la modification dirigée des acides aminés le constituant est susceptible d'entraîner une amélioration de l'activité. Ainsi, il est proposé dans le brevet européen No. 527 809 de remplacer séquentiellement les acides aminés d'une région active par un acide aminé fréquemment impliqué dans les sites actifs (la sérine par exemple) en utilisant une technique apparentée à la mutagenèse dirigée. Cette technique présuppose cependant de disposer d'informations précises concernant le site actif de la molécule étudiée, et utilise une technologie ne permettant pas d'introduire un grand nombre de modifications sur un fragment d'ADN.

A l'inverse, des expériences de mutagenèse aléatoire ont été menées dans un contexte de recherche de perte d'activité (Loeb et al., 1989, Nature, 340 :397). Dans ce cas, de très nombreux clones doivent être analysés pour obtenir des résultats concordants et ainsi s'affranchir des limites posées par le remplacement aléatoire.

La présente invention est une technique intermédiaire entre la mutagenèse dirigée et la mutagenèse aléatoire. En effet, son objet n'est pas comme dans le cas de la mutagenèse dirigée simple d'obtenir un seul produit en fin de réaction contenant la ou les mutations souhaitées, mais d'obtenir un mélange de molécules contenant chacune une ou plusieurs des mutations désirées. L'objet du procédé objet de la présente invention n'est pas non plus d'introduire au niveau d'une position donnée n'importe quelle substitution, comme dans le cas de la mutagenèse aléatoire, mais au contraire d'y introduire un type de substitution particulier et prédéfini.

Ce procédé combine ainsi les avantages de la mutagenèse dirigée (contrôle de la nature des modifications obtenues en une position donnée), et de la mutagenèse aléatoire (obtention d'un grand nombre de mutations différentes réparties sur de nombreuses positions du fragment d'ADN à muter). Il permet de réaliser en un temps très court un grand nombre de mutations dirigées.

Ces buts sont atteints selon l'invention grâce à un procédé de mutagenèse d'un gène cible consistant à préparer une série de N oligonucléotides de séquence substantiellement complémentaire d'au moins une région du gène cible, puis à faire réagir ladite série d'oligonucléotide avec ledit gène cible dans des conditions permettant la production de copies du gène cible portant au moins une mutation. Le gène cible est porté par un plasmide circulaire double-brin. Chaque oligonucléotide présente une séquence complémentaire d'une région différente du gène cible et au moins une mutation placée au centre de la séquence de l'oligonucléotide. l'ensemble N desdits oligonucléotides de la série, avec N supérieur à 5, couvre tout ou partie de la séquence dudit gène cible. On fait ensuite réagir ladite série d'oligonucléotides avec le gène cible en présence d'une polymérase de façon à générer une banque de gènes mutés où chaque mutation provenant d'un oligonucléotide différent est présente en moyenne dans moins de 1/5 des gènes de la banque.

Le procédé de l'invention est remarquable en ce que la contrainte liée à la position de la mutation et celle liée à sa nature sont dissociées ; il est par exemple possible de réaliser des mutations d'un type particulier (par exemple n'importe quel codon vers Alanine) sur toute une séquence codante. Dans cet exemple, chacun des mutants obtenus en fin de réaction contient une ou plusieurs mutations de nature connue (codon alanine), mais de position non connue. A l'inverse, le procédé de l'invention permet d'incorporer sur quelques positions particulières des oligonucléotides contenant des bases dégénérées. Dans cet exemple, les positions sont relativement connues (le nombre de positions est limité), et la nature des mutations introduites est inconnue.

Ces caractéristiques contrastent par rapport aux techniques de mutagenèse aléatoire, où ni la nature ni la position des mutations ne sont connues, et la mutagenèse dirigée classique, où la nature et la position de la mutation introduite sont, connues.

Le procédé de l'invention est également remarquable par le fait qu'à l'issue de la réaction de mutagenèse, on dispose d'un grand nombre de molécules d'ADN différentes, constituant donc une banque. Ces molécules correspondent à toutes les molécules d'ADN ayant incorporé au moins une mutation au niveau des sites préalablement pointés. Le nombre de molécules mutantes différentes est très important car toutes les combinaisons de mutations sont possibles.

Le procédé de mutagenèse selon l'invention peut également s'appliquer à une banque de gènes mutés. Cette banque est alors utilisée comme matrice à la place du gène cible. On prépare une série de N oligonucléotides de séquence substantiellement complémentaire d'au moins une région des gènes mutés cibles, puis on fait réagir ladite série d'oligonucléotides avec lesdits gènes mutés cibles dans des conditions permettant la production de copies des gènes mutés cibles portant au moins une mutation. Les gènes mutés cibles sont portés par des plasmides circulaires double brin, et chaque oligonucléotide présente une séquence complémentaire d'une région différente des gènes mutés cibles et au moins une mutation placée au centre de la séquence de l'oligonucléotide, l'ensemble N desdits oligonucléotides de la série, avec N supérieur à 5, couvre tout ou partie de la séquence desdits gènes mutés cibles. On fait ensuite réagir ladite série d'oligonucléotides avec les gènes mutés cibles en présence d'une polymérase de façon à générer une banque de gènes mutés où chaque mutation provenant d'un oligonucléotide différent est présente en moyenne dans moins de 1/5 des gènes de la banque.

Une autre application du procédé selon l'invention consiste en ce que la banque de gènes mutés cibles utilisée comme matrice a préalablement été obtenue par le procédé de mutagenèse massive. Le nombre moyen de mutations par molécule augmente avec le nombre de tours de mutagenèse massive réalisés.

Selon une forme avantageuse de réalisation du procédé de l'invention, N est compris entre environ 5 et 10⁶ et de préférence entre 50 et 500, et chaque mutation provenant d'un oligonucléotide différent est présente en moyenne dans entre 1/5 et 1/10⁶ et de préférence entre 1/50 et 1/500 des gènes de la banque.

Le procédé de l'invention envisage tout particulièrement le cas où la série d'oligonucléotides comprend N oligonucléotides différents et où chaque mutation provenant d'un oligonucléotide différent est présente dans en moyenne environ 1/N des gènes de la banque, avec N ayant la valeur ci-dessus.

Cette caractéristique distingue le procédé de l'invention de l'art antérieur, où les exemples de mutagenèse multiple utilisant simultanément plusieurs oligonucléotides sont au contraire basées sur des taux d'incorporation de chaque oligonucléotide supérieur à 75%. En effet, ces approches ont pour but d'isoler uniquement le mutant ayant incorporé tous les oligonucléotides, et les taux d'incorporation élevés permettent facilement de parvenir à ce but. Au contraire, dans le procédé selon l'invention, la fréquence de mutation au niveau de chacune des mutations à introduire est contrôlée de façon à ne pas obtenir de molécules d'ADN contenant un trop grand nombre de mutations. L'objectif est de disposer de mutants contenant chacun une mutation ou une combinaison de quelques mutations différentes. Pour ce faire, le ratio entre la quantité de chaque oligonucléotide mutant et la quantité de matrice à muter doit être contrôlé. Ce ratio est compris entre 0,01 et 100, et préférentiellement entre 0,1 et 10 selon les cas.

La réaction entre la série d'oligonucléotides avec le gène cible ou avec la banque de gènes mutés cibles peut être réalisée avec différents types de polymérase, avantageusement thermostables. Un premier mode de réalisation consiste à utiliser une polymérase ayant une activité de déplacement de brin comme la Taq polymérase, ou une activité exonucléasique 3'-> 5' comme la Pfu polymérase. Dans ce mode de réalisation, la réaction peut comprendre la présence d'une ligase. Un second mode de réalisation consiste à utiliser une polymérase n'ayant pas d'activité de déplacement de brin ou exonucléasique 3'-> 5' comme la T4 polymérase, et dans ce cas la réaction est réalisée en l'absence de ligase.

Les oligonucléotides de la série ont une taille comprise entre 10 et 100 et de préférence entre 15 et 25 nucléotides. Chacun d'entre eux est homologue d'une partie dé la séquence d'ADN à muter, à l'exclusion d'une ou de quelques positions localisées dans sa partie interne, qui constituent la ou les mutations à introduire. Ces oligonucléotides peuvent être chevauchants, c'est-à-dire comprendre des séquences communes de deux régions différentes adjacentes. Les oligonucléotides sont préférentiellement tous de même orientation, de façon à ce qu'un seul brin du gène cible soit répliqué, ce qui permet d'obtenir un taux de mutagenèse faible.

Dans un mode de réalisation particulier, les oligonucléotides sont reconstruits à partir de deux oligonucléotides, selon le procédé décrit dans le brevet US 5 858 731.

Avantageusement, chaque oligonucléotide comprend 1 ou plus mutation(s) et de préférence de 1 à 3 mutation(s) placée(s) au centre de sa séquence.

Lesdites mutations de chaque oligonucléotide peuvent être choisies parmi des délétions et/ ou des insertions de un ou plusieurs nucléotide(s).

Une forme particulière de mutations consiste à utiliser des oligonucléotides dégénérés. C'est-à-dire que chaque oligonucléotide de la série est présent en plusieurs exemplaires, chaque exemplaire possédant un nucléotide différent au niveau de la ou desdites mutation(s).

Une autre forme particulière de mutation consiste en ce que chaque mutation est du type permettant d'introduire un codon identique dans chaque oligonucléotide ou un codon correspondant au même acide aminé, en substitution du codon original du gène cible. Avantageusement, ledit codon correspond à un acide aminé choisi dans le groupe comprenant Ala, Val, Gly, Leu, Ile.

Le procédé de l'invention peut être décrit plus précisément à l'aide des étapes suivantes :
- On prépare la matrice, de préférence un plasmide contenant le ou les fragments d'ADN à muter.
- On synthétise les oligonucléotides mutants différents de préférence entre 5 et 10⁶, et plus préférentiellement entre 50 et 500, puis on mélange l'ensemble des oligonucléotides mutants. La concentration finale de chacun des oligonucléotides dans le mélange est ainsi divisée lors de cette étape par le nombre d'oligonucléotides.
- On les ajoute à la matrice, c'est-à-dire au plasmide contenant le ou les fragments d'ADN à muter, à une concentration telle que le rapport entre le nombre de molécules de matrice et le nombre de molécules de chacun des oligonucléotides mutants soit compris entre 0,01 et 100, et de préférence entre 0,1 et 10.
- On dénature la matrice par la chaleur (environ 95°C), de façon à disposer temporairement d'ADN simple brin. Lors du retour à une température plus basse, certains ou tous les oligonucléotides présents dans le mélange se fixent sur la matrice au niveau de leur site d'homologie.
- On ajoute tous les éléments nécessaires à réaliser une réplication de la matrice à partir des oligonucléotides mutants, et notamment une polymérase, le tampon permettant son activité, des nucléotides triphosphates en quantité suffisante, les éventuels cofacteurs nécessaires. La réaction de réplication a ensuite lieu dans les conditions de température correspondant à l'activité maximale de la polymérase. Éventuellement, une ligase peut être ajoutée lors de l'étape de réplication, de façon à ce que les brins d'ADN néosynthétisés se liguent au niveau de l'extrémité 5' d'un autre oligonucléotide, lié sur la matrice en 3' du premier. Dans ce cas, les oligonucléotides sont préalablement phosphorylés.
- Éventuellement, on répète les étapes de dénaturation et de réplication plusieurs fois. Dans ce cas, il est préférable que la polymérase soit thermostable, afin d'éviter la nécessité de rajouter de l'enzyme à chaque cycle. A l'issue dé cette réaction, les molécules d'ADN présentes dans le mélange sont de plusieurs types :

- d'une part, il reste de la matrice initiale double brin n'ayant pas été efficacement répliquée,
- d'autre part, une partie des molécules a été répliquée, c'est-à-dire qu'elles contiennent un brin d'origine et un brin néosynthétisé à partir d'une ou de plusieurs amorces que constituent les oligonucléotides mutants.
- On soumet le mélange obtenu à la digestion par l'enzyme DpnI, ou une autre enzyme de restriction permettant de supprimer les molécules d'ADN méthylés sur les deux brins et de conserver celles qui ne sont pas méthylés ou hémiméthylés.
- On transforme des bactéries compétentes par le mélange précédent, ces bactéries sont ensuite étalées sur un milieu contenant un agent sélectif de façon à sélectionner les bactéries ayant intégré un plasmide. Les molécules d'ADN qui ont été clivées à l'étape précédente sont éliminées car la présence d'un plasmide circulaire complet est indispensable à la survie de la bactérie en milieu sélectif.
- On prélève individuellement les colonies bactériennes obtenues et on les utilise pour ensemencer un milieu nutritif sélectif. Une préparation d'ADN plasmidique de ces cultures est ensuite réalisée afin d'isoler une grande quantité d'ADN plasmidique potentiellement mutant. Un examen des différentes molécules d'ADN plasmidiques à ce stade permet de calculer le taux moyen d'incorporation de chaque oligonucléotide, et de vérifier que celui-ci est voisin de la valeur recherchée.
- Éventuellement, on teste l'activité biologique correspondant aux différents lots de préparations plasmidiques mutantes. L'activité mesurée peut être supérieure à celle du fragment non muté, égale, ou inférieure. Dans le cas où l'activité est modifiée, le plasmide correspondant est séquencé de façon à pouvoir localiser la position de la mutation introduite. Dans un mode de réalisation préféré, on teste l'activité biologique correspondant aux molécules mutantes, et on compare cette mesure à celle de l'activité biologique correspondant à la molécule d'ADN plasmidique non mutée. Lorsque l'observation de ces mesures fait apparaître une différence significative, les molécules mutantes sont séquencées afin de mettre en évidence la position de la mutation à l'origine de cette modification d'activité. L'ordre de ces deux dernières étapes est inversé par rapport aux techniques habituelles de mutagenèse dirigée, qui impliquent généralement l'obtention et la vérification de la molécule mutante préalablement au test de son activité biologique.

Ainsi, une forme de réalisation du procédé de mutagenèse selon l'invention comprend les étapes suivantes :
a) on prépare une matrice constituée d'un plasmide contenant le gène cible ou la banque de gènes mutés cibles et un gène de résistance,
b) on prépare une série équimoléculaire d'oligonucléotides présentant une séquence complémentaire d'une région différente du gène cible ou la banque de gènes mutés cibles et au moins une mutation placée au centre de la séquence de l'oligonucléotide, l'ensemble des oligonucléotides de la série couvrant tout ou partie de la séquence dudit gène cible,
c) on mélange la série d'oligonucléotides préparée à l'étape (b) avec le plasmide de l'étape (a) selon un rapport molaire de chaque oligonucléotide par rapport au plasmide compris entre 0,01 et 100 et de préférence entre 0,1 et 10,
d) on dénature le mélange de l'étape (c) par la température de façon à obtenir une matrice simple brin,
e) on soumet le mélange de l'étape (d) à une température permettant l'hybridation des oligonucléotides sur la matrice,
f) on ajoute au mélange au moins une polymérase, son tampon et ses cofacteurs, et une quantité suffisante de chacun des nucléotides triphosphates, pour permettre la réplication des brins de la matrice à partir de n'importe lequel des oligonucléotides,
g) éventuellement on répète les étapes (d), (e) et (f),
h) on sélectionne par tout moyen approprié les produits de l'étape (f) ou (g) ayant subi la réplication,
i) on transforme les produits sélectionnés à l'étape (h) dans des bactéries compétentes, et l'on sélectionne les produits portant un plasmide sur un milieu sélectif correspondant à un gène de résistance porté par le plasmide de l'étape (a).
   Avantageusement, l'étape (h) ci-dessus consiste à soumettre les produits de l'étape (f) à l'action d'une enzyme de restriction sélectionnant les produits ayant subi la réplication, comme l'enzyme DpnI.
   Les oligonucléotides peuvent être phosphorylés à leur extrémité 5' lorsqu'à l'étape (f) on ajoute une ligase, avantageusement une ligase thermostable.
   Le procédé de mutagenèse selon l'invention est particulièrement utile pour mesurer l'activité biologique des protéines mutées codées par les gènes cibles mutés. En conséquence, le gène cible est une molécule d'acide nucléique codant pour une protéine d'intérêt avec ou sans ses propres séquences de régulation. Si le gène cible ne comprend pas les séquences de régulation, comme un promoteur, celles-ci sont présentes au niveau du plasmide.
   L'invention concerne donc aussi un procédé de mutagenèse d'une protéine cible ou d'une banque de protéines mutées cibles, caractérisé en ce qu'il comprend la préparation d'une banque d'expression de gènes mutés à partir d'un gène cible codant pour ladite protéine selon le procédé de mutagenèse décrit précédemment, puis l'expression desdits gènes mutés pour produire une banque de protéines mutées, et éventuellement le criblage desdites protéines mutées pour une fonction désirée, avantageusement par rapport à la protéine cible.
   L'invention permet donc la réalisation d'un procédé de sélection de protéines mutées présentant une activité modifiée par rapport à la même protéine non mutée, ou du gène muté correspondant à ladite protéine mutée, comprenant un procédé de mutagenèse ci-dessus, puis après le criblage desdites protéines mutées pour une fonction désirée, avantageusement par rapport à la protéine cible, la sélection de la protéine mutée présentant la fonction désirée, et éventuellement le séquençage du gène muté correspondant à ladite protéine mutée.
   Ces procédés de mutagenèse d'une protéine cible ou d'une banque de protéines mutées cibles ou de sélection d'une protéine mutée ou du gène correspondant selon l'invention sont caractérisés en ce qu'ils comprennent la préparation d'une banque d'expression de gènes mutés à partir d'un gène cible codant pour ladite protéine, puis les étapes suivantes :
j) on incube le milieu sélectif à la température adéquate pendant le temps suffisant à la pousse de colonies bactériennes individuelles,
k) on ensemence des cultures de bactéries individuelles à partir des colonies de l'étape (j),
l) on effectue des préparations d'ADN plasmidique des cultures de l'étape (k),
m) on mesure l'activité biologique associée à chaque préparation d'ADN plasmidique de l'étape (1) et on compare le résultat obtenu par rapport à celui mesuré en utilisant l'ADN plasmidique non muté.
n) éventuellement, on séquence les préparations d'ADN plasmidique ayant permis d'observer des modifications significatives de l'activité biologique.

L'invention a également pour objet une série d'oligonucléotides mutés par rapport à un gène cible ou à la banque de gènes mutés cibles comme défini précédemment.

L'invention a encore pour objet une banque de gènes mutés susceptibles d'être obtenue par un procédé décrit précédemment caractérisée en ce que chaque mutation différente est présente en moyenne dans moins de 1/5 des gènes de la banque et de préférence en moyenne dans environ 1/N des gènes de la banque, avec N supérieur à 5, de préférence avec N compris entre environ 5 et 10⁶ et tout préférentiellement avec N compris entre 50. et 500. De préférence, cette banque est obtenue, conformément au procédé de l'invention, grâce à la mise en oeuvre de N oligonucléotides avec N supérieur à 5, de préférence avec N compris entre environ 5 et 10⁶ et tout préférentiellement avec N compris entre 50 et 500.

D'autres avantages et caractéristiques de l'invention apparaîtront des exemples qui suivent et des dessins en annexe dans lesquels :

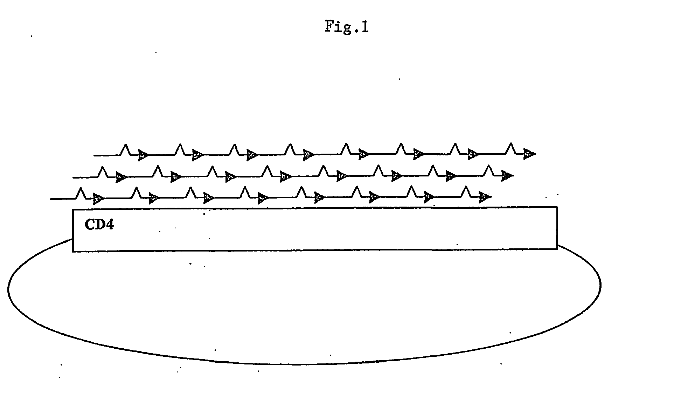

La figure 1 représente une répartition des oligonucléotides mutants sur la séquence de la molécule CD4. Chacun des oligonucléotides contient trois mutations au maximum, représentées ici par la brisure de la ligne de la flèche, localisées en son centre, destinées à changer le codon visé en Alanine. Les oligonucléotides se chevauchent les uns les autres, et sont représentés ici sur trois niveaux par mesure de clarté. Dans l'exemple de la figure 1, 24 oligonucléotides mutants seulement sont représentés. Dans l'exemple 1 ci-après, 95 oligonucléotides ont été utilisés simultanément. Dans cet exemple, les oligonucléotides portant les mutations sont tous de même orientation.

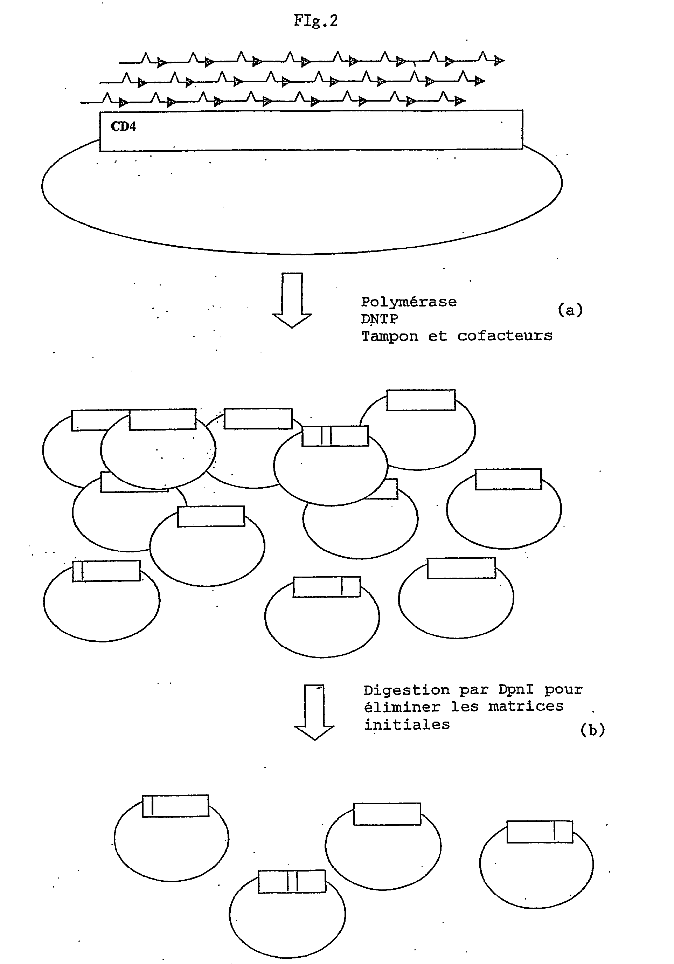

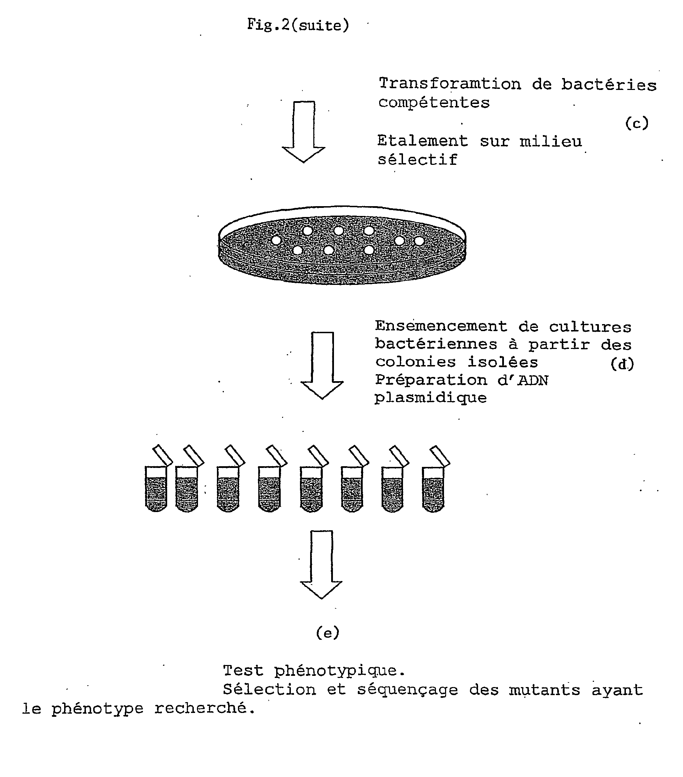

La figure 2 est un résumé schématique des étapes du procédé de mutagenèse selon l'invention à partir des oligonucléotides mutants de la figure 1. Les mutations dans les molécules d'ADN sont représentées par une barre verticale. Les lettres signifient :
   - (a) polymérisation (polymérase, dNTP, Tampon et cofacteurs) ;
   - (b) digestion par DpnI pour éliminer les matrices initiales ;
   - (c) transformation de bactéries compétentes puis étalement sur milieu sélectif ;
   - (d) ensemencement de cultures bactériennes à partir des colonies isolées puis préparation d'ADN plasmidique.
   - (e) test phénotypique puis sélection et séquençage des mutants ayant le phénotype recherché.
La figure 3 représente un exemple de construction d'oligonucléotides mutants à partir de demi-oligonucléotides comprenant une ligation par la T4 ligase.
La figure 4 donne des exemples de représentation schématique des oligonucléotides contenant des bases dégénérées au niveau des points de mutation. La lettre N signifie que n'importe laquelle des 4 bases peut être présente à ce endroit. Ainsi l'oligonucléotide concerné est en réalité constitué d'un mélange de 4^{N} espèces moléculaires.

### Exemple 1 : Alanine Scanning.

Un gène codant pour la molécule CD4 a été introduit dans le vecteur SK+. Les 95 premiers codons de ce gène ont été la cible d'une réaction de mutagenèse massive selon la technique de l'invention.

Pour ce faire, une série de 95 oligonucléotides de 21 bases ont été synthétisés. Chacun de ces oligonucléotides était complémentaire d'une séquence du gène CD4 centrée sur un codon. Ainsi; le premier oligonucléotide était homologue d'une séquence centrée sur le codon. 1. Il était parfaitement homologue à 9 bases de chaque côté dudit codon, et contenait 3 mutations, destinées à transformer le premier codon en codon Alanine (Figure 1).

De même, le second oligonucléotide était centré sur le second codon et contenait trois mutations adjacentes en son milieu.

Les 95 oligonucléotides, chevauchants entre eux et tous de même orientation, étaient ensuite mélangés de façon équimolaire et phosphorylés en utilisant la kinase de T4 dans des conditions standards d'utilisation.

La réaction suivante a ensuite été réalisée :

| | |
|---|---|
| Matrice SK-CD4 (250 µg/ µl) : | 1 µl |
| MIX des 95 oligonucléotides 5'P (0,5 µM chacun) | 2 µl |
| dNTP Triphosphates (2,5 mM) | 10 µl |
| Tampon 10X de Pfu Polymérase | 2,5 µl |
| ATP 10 mM | 0,5 µl |
| Pfu Polymérase (2,5 U / µl) | 1 µl |
| Pfu Ligase (4 U / µl) | 1 µl |
| H₂O | 7 µl |
| Total | 25 µl |

Le mélange a été soumis à une réaction de 12 cycles de température [(94°C, 1') ;(35°C, 1') ; (68°C, 20')].

Le mélange réactionnel a ensuite été soumis à une digestion par 5 unités de l'enzyme DpnI dans des conditions de tampon adaptées, pendant 30' à 37°C.

Des bactéries compétentes ont été transformées par ce mélange en utilisant un protocole de choc thermique, et étalées sur une boite de pétri contenant l'agent sélectif.

Le lendemain, un millier de bactéries étaient obtenues.

Un schéma résumant les étapes de la technique est proposé en figure 2.

A ce stade, un test statistique de quelques molécules d'ADN de la banque a été réalisé pour mesurer le taux d'intégration des mutations, c'est-à-dire le remplacement d'un des 95 codons par un codon Alanine. Cet examen révélait que la fréquence d'incorporation de chaque oligonucléotide était voisine de 1%, ce qui était dans ce cas la valeur recherchée (1/N avec N=95).

Les molécules mutantes ont ensuite été amplifiées par culture bactérienne et préparation d'ADN plasmidique. Chacun des lots d'ADN plasmidique, correspondant à un type unique de molécule mutante, a été utilisé pour transfecter des cellules eucaryotes, et celles-ci ont été testées pour le maintien ou la perte des épitopes portés par la molécule CD4, cette activité étant mesurée par la liaison d'un anticorps anti-CD4.

### Exemple 2 : Valine scanning en utilisant la reconstruction des oligonucléotides reconstruits à partir de demi-oligonucléotides.

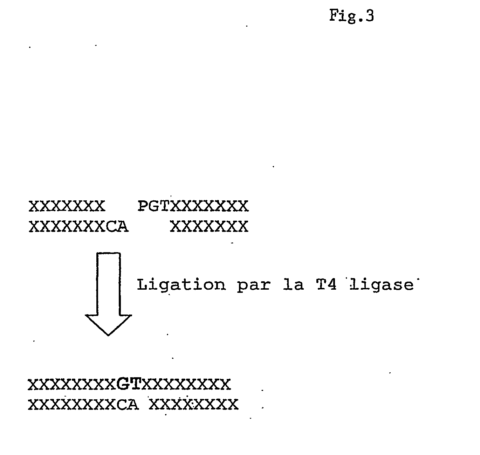

Une série de 11 oligonucléotides ont été chacun reconstruits à partir de deux demi-oligonucléotides double brin. La ligation des deux demi-oligonucléotides a été effectuée par une réaction utilisant la T4 ligase. Seul un des deux demi-oligonucléotides étant phosphorylé au niveau d'une de ses extrémités 5', cette réaction avait pour effet de permettre l'obtention d'un seul oligonucléotide complet, composé de 18 bases (8 de chaque côté parfaitement homologues, et deux en son milieu invariablement constituées des nucléotides GT (figure 3).

Ces mutations, destinées à remplacer les deux premiers nucléotides de n'importe quel codon, permettent de remplacer ce codon par un codon Valine. En effet, le code génétique étant dégénéré, les codons valine peuvent s'écrire sous la forme GTN, où N représente n'importe laquelle des quatre bases.

Une fois obtenus ces 11 oligonucléotides reconstruits, homologues de 11 régions distinctes de la molécule CD4, mais portant la même mutation (changement en Valine), par ailleurs orientés dans le même sens, le protocole était identique à celui exposé dans l'exemple 1.

Il était recherché une fréquence moyenne d'incorporation des oligonucléotides mutants d'environ 9% (1/11) dans cet exemple.

### Exemple 3 Amélioration d'un site actif par mutagenèse massive à saturation.

Une fois connus les acides aminés prenant part directement au site actif d'une protéine, par exemple grâce à des données de mutagenèse massive telle que présentée dans les exemple précédents, ces acides aminés peuvent être soumis à une mutagenèse massive à saturation, c'est-à-dire visant à introduire un grand nombre de codons différents en remplacement d'un codon particulier. Ainsi, 6 codons du gène codant pour la protéine agaB, enzyme impliquée dans la synthèse des sucres, étaient identifiés comme prenant part directement à l'activité de l'enzyme.

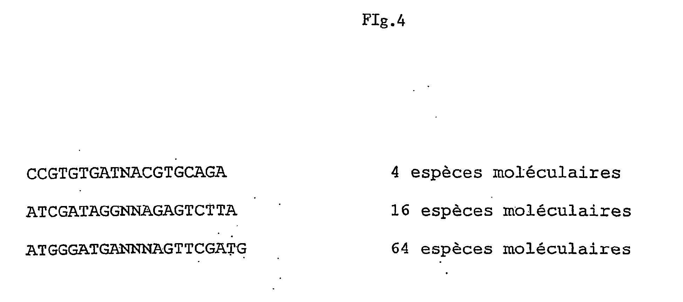

Six oligonucléotides centrés sur ces codons étaient synthétisés. De chaque côté, ils étaient totalement homologues à 9 bases de la séquence. Au niveau des deux premiers nucléotides des codons à muter, la séquence sauvage était remplacée par la séquence NN(figure 4). De cette façon, chacun des 6 codons pouvait être remplacé par différents codons spécifiques d'autres acides aminés.

Une fois obtenus ces oligonucléotides, la réaction de mutagenèse massive était identique à celle décrite dans l'exemple 1.

I1 était recherché une fréquence moyenne d'incorporation des oligonucléotides mutants d'environ 17% (1/6) dans cet exemple.

Ces molécules d'ADN plasmidique étaient destinées à être criblées de façon à mesurer une augmentation de l'activité de l'enzyme.

Cet exemple, bien que n'utilisant qu'un nombre réduit d'oligonucléotides mutants contenant des bases dégénérées, démontre la faisabilité de l'adaptation de la technique de l'invention à la mutagenèse de nature aléatoire (changement d'un codon en un grand nombre d'autres codons) mais de positions déterminées.

### Exemple 4 : Optimisation des codons d'un gène.

Les gènes contiennent généralement des codons qui sont défavorables à l'expression des protéines pour lesquelles ils codent. Ces codons défavorables peuvent être vus comme un mécanisme de régulation de l'expression d'un gène. Ces codons défavorables sont relativement bien identifiés, et il peut être nécessaire de les modifier en un codon plus favorable à l'expression de la protéine mais n'induisant pas de changement d'acide aminé.

En règle générale, environ 5 % des codons d'un gène sont défavorables, et limitent le niveau d'expression de la protéine correspondante. La modification de ces codons peut permettre d'obtenir de meilleurs niveaux d'expression du gène lors de la production *in vitro* de la protéine correspondante.

Pour autant, la modification simultanée de tous les codons ne permet pas d'obtenir cette amélioration de niveau d'expression, probablement à cause de la déstabilisation générale de la séquence par un trop grand nombre de modifications.

Les meilleurs niveaux d'expression sont le plus souvent obtenus lorsqu'une partie seulement des codons défavorables sont modifiés.

Dans ce cas, une banque de mutations portant sur ces codons défavorables peut être réalisée en utilisant la technique de l'invention, et les molécules mutantes présentant les meilleurs taux d'expression sont alors sélectionnées. Pour ce faire, un oligonucléotide mutant doit être synthétisé pour chaque codon défavorable, les mutations portées par ces oligonucléotides étant définies par le fait qu'elles changent un codon défavorable à l'expression en codon lui étant favorable, sans changer la séquence primaire de la protéine correspondante.

Dans ce contexte, la fréquence moyenne d'incorporation des oligonucléotides mutants peut être recherchée dans une fourchette relativement large, par exemple entre 1 et 20 %. En effet, le nombre de mutations simultanées permettant d'obtenir le meilleur niveau d'expression n'est pas connu. Dans ce contexte, plusieurs banques correspondant à des fréquences d'incorporation différentes peuvent être réalisées. Pour réaliser ces banques ayant des taux de mutations différents, il est possible soit de réaliser le procédé décrit en utilisant des concentrations variables d'oligonucléotides, soit de réaliser plusieurs fois de suite le procédé de mutagenèse massive, c'est-à-dire d'utiliser la banque de mutants produite au cours d'une première étape de mutagenèse massive comme matrice d'une seconde étape de mutagenèse massive.

Le procédé est similaire à celui présenté dans l'exemple 1. Les molécules mutantes sont ensuite criblées sur le critère de leur niveau d'expression : les molécules présentant les meilleurs niveaux d'expression sont sélectionnées.

## Revendications

1. procédé de mutagenèse d'un gène cible, consistant à préparer une série de N oligonucléotides, chaque oligonucléotide présentant une séquence complémentaire d'une région différente du gène cible et de une à trois mutations placées au centre de la séquence de l'oligonucléotide, puis à faire réagir ladite série d'oligonucléotides avec ledit gène cible dans des conditions permettant la production de copies du gène cible portant au moins une mutation, **caractérisé en ce que** le gène cible est porté par un plasmide circulaire double brin, l'ensemble N desdits oligonucléotides de la série, avec N supérieur à 5, couvrant tout ou partie de la séquence dudit gène cible, et **en ce que** l'on fait réagir ladite série d'oligonucléotides avec le gène cible en présence d'une polymérase de façon à générer après sélection par un enzyme de restriction permettant de supprimer les molécules d'ADN méthylées sur les deux brins et de conserver celles qui ne sont pas méthylées ou hémiméthylées, une banque de gènes mutés où chaque mutation provenant d'un oligonucléotide différent est présente en moyenne dans moins de 175 des gènes de la banque.

2. Procédé de mutagenèse d'une banque de gènes mutés cibles, consistant à préparer une série de N oligonucléotides, chaque oligonucléotide présentant une séquence complémentaire d'une région différente des gènes mutés cibles et de une à trois mutations placées au centre de la séquence de l'oligonucléotide, puis à faire réagir ladite série d'oligonucléotides avec lesdits gènes mutés cibles dans des conditions permettant la production de copies des gènes mutés cibles portant au moins une mutation, **caractérisé en ce que** les gènes mutés cibles sont portés par des plasmides circulaires double brin, l'ensemble N desdits oligonucléotides de la série, avec N supérieur à 5, couvrant tout ou partie de la séquence desdits gènes mutés cibles, et **en ce que** l'on fait réagir ladite série d'oligonucléotides avec les gènes mutés cibles en présence d'une polymérase de façon à générer après sélection par un enzyme de restriction permettant de supprimer les molécules d'ADN méthylées sur les deux brins et de conserver celles qui ne sont pas méthylées ou hémiméthylées, une banque de gènes mutés où chaque mutation provenant d'un oligonucléotide différent est présente en moyenne dans moins de 1/5 des gènes de la banque.

3. Procédé de mutagenèse selon la revendication 2, dans lequel la banque de gènes mutés cibles a été obtenue selon le procédé de la revendication 1.

4. Procédé de mutagenèse selon les revendications 1 à 3, **caractérisé en ce que** N est compris entre 5 et 10⁶, et **en ce que** chaque mutation provenant d'un oligonucléotide différent est présente en moyenne dans entre 1/5 et 1/10⁶.

5. Procédé de mutagenèse selon l'une des revendications 1 à 4, **caractérisé en ce que** la série d'oligonucléotides comprend N oligonucléotides différents et **en ce que** chaque mutation provenant d'un oligonucléotide différent est présente dans en moyenne environ 1/N des gènes de la banque.

6. Procédé de mutagenèse selon l'une quelconque des revendications 1 à 5, **caractérisé en ce que** l'on fait réagir la série d'oligonucléotides avec le gène cible ou la banque de gènes mutés cibles selon un rapport molaire de chaque oligonucléotide par rapport au gène cible ou à la banque de gènes mutés cibles compris entre 0,01 et 100.

7. Procédé de mutagenèse selon l'une quelconque des revendications 1 à 6, **caractérisé en ce que** l'on fait réagir la série d'oligonucléotides avec le gène cible ou la banque de gènes' mutés cibles en présence d'une polymérase ayant une activité de déplacement de brin comme la Taq polymérase ou une activité exonucléasique 3'→ 5' comme la Pfu polymérase.

8. Procédé de mutagenèse selon la revendication 7, **caractérisé en ce que** l'on fait réagir la série d'oligonucléotides avec le gène cible ou la banque de gènes mutés cibles en présence d'une polymérase ayant une activité de déplacement de brin ou une activité exonucléasique 3'→ 5' et une ligase.

9. Procédé de mutagenèse selon l'une quelconque des revendications 1 à 6, **caractérisé en ce que** l'on fait réagir la série d'oligonucléotides avec le gène cible ou la banque de gènes mutés cibles en présence d'une polymérase n'ayant pas d'activité de déplacement de brin ou d'activité exonucléasique 3' -> 5', comme la T4 polymérase, et en l'absence de ligase.

10. Procédé de mutagenèse selon l'une quelconque des revendications précédentes, **caractérisé en ce que** les oligonucléotides de la série sont chevauchants ou non et présentent une taille comprise entre 10 et 100.

11. Procédé de mutagenèse selon l'une quelconque des revendications précédentes, **caractérisé en ce que** chaque oligonucléotide comprend de 1 à 3 mutation(s) placée (s) au centre de sa séquence.

12. Procédé selon l'une quelconque des revendications précédentes, **caractérisé en ce que** les mutations de chaque oligonucléotide sont choisies parmi des délétions et ou des insertions de un ou plusieurs nucléotide (s).

13. Procédé de mutagenèse selon l'une quelconque des revendications précédentes, **caractérisé en ce que** chaque oligonucléotide de la série est présent en plusieurs exemplaires chaque exemplaire possédant un nucléotide différent au niveau de la ou desdites mutation(s).

14. Procédé de mutagenèse selon l'une quelconque des revendications précédentes, **caractérisé en ce que** chaque mutation est du type permettant d'introduire un codon identique dans chaque oligonucléotide ou un codon correspondant au même acide aminé, en substitution du codon original du gène cible.

15. Procédé de mutagenèse selon la revendication 14, **caractérisé en ce que** ledit codon correspond à un acide aminé choisi dans le groupe comprenant Ala, Val, Gly, Leu, Ile.

16. Procédé de mutagenèse selon l'une quelconque des revendications 1 à 15, **caractérisé en ce qu'**il comprend les étapes suivantes :
a) on prépare une matrice constituée d'un plasmide contenant le gène cible ou la banque de gènes mutés cibles et un gène de résistance,
b) on prépare une série équimoléculaire d'oligonucléotides présentant une séquence complémentaire d'une région différente du gène cible ou de la banque de gènes mutés cibles et au moins une mutation placée au centre de la séquence de l'oligonucléotide, l'ensemble des oligonucléotides de la série couvrant tout ou partie de la séquence dudit gène cible ou de ladite banque de gènes mutés cibles,
c) on mélange la sérié d'oligonucléotides préparée à l'étape (b) avec le plasmide de l'étape (a) selon un rapport molaire de chaque oligonucléotide par rapport au plasmide compris entre 0,01 et 100
d) on dénature le mélange de l'étape (c) par la température de façon à obtenir une matrice simple brin,
e) on soumet le mélange de l'étape (d) à une température permettant l'hybridation des oligonucléotides sur la matrice,
f) on ajoute au mélange une polymérase, son tampon et ses cofacteurs, et une quantité suffisante de chacun des nucléotides triphosphates, pour permettre la réplication des brins de la matrice à partir dé n'importe lequel des oligonucléotides,
g) éventuellement on répète les étapes (d), (e) et (f),
h) on sélectionne par tout moyen approprié les produits dé l'étape (f) ou (g) ayant subi la réplication,
i) on transforme les produits sélectionnés à l'étape (h) dans des bactéries compétentes, et l'on sélectionne les produits portant un gène muté sur un milieu sélectif correspondant à un gène de résistance porté par le plasmide de l'étape (a).

17. Procédé de mutagenèse selon la revendication 16, **caractérisé en ce que** l'étape (h) consiste à soumettre les produits de l'étape (f) à l'action d'une enzyme de restriction sélectionnant les produits ayant subi la réplication.

18. Procédé de mutagenèse selon la revendication 17, **caractérisé en ce que** l'enzyme de restriction sélectionnant les produits ayant subi la réplication est l'enzyme DpnI.

19. Procédé de mutagenèse selon l'une quelconque des revendications 16 à 18, **caractérisé en ce que** les oligonucléotides sont phosphorylés à leur extrémité 5' et **en ce qu'**à l'étape (f) on ajoute une ligase.

20. Procédé de mutagenèse selon l'une Quelconque des revendications précédentes, **caractérisé en ce que** le gène cible est une molécule d'acide nucléique codant pour une protéine d'intérêt avec ou sans ses propres séquences de régulation.

21. Procédé de mutagenèse d'une protéine cible ou d'une banque de protéines mutées cibles, **caractérisé en ce qu'**il comprend la préparation d'une banque d'expression de gènes mutés à partir d'un gène cible codant pour ladite protéine selon l'une quelconque des revendications 1 à 20, puis **en ce que** l'on exprime lesdits gènes mutés pour produire une banque de protéines mutées, et éventuellement en ce que l'on crible lesdites protéines mutées pour une fonction désirée.

22. Procédé de sélection de protéines mutées présentant une activité modifiée par rapport à la même protéine non mutée, ou du gène muté correspondant à ladite protéine mutée, **caractérisé en ce qu'**il comprend un procédé de mutagenèse selon la revendication 21, **en ce qu'**après le criblage desdites protéines mutées pour une fonction désirée, on sélectionne la protéine mutée présentant la fonction désirée, et éventuellement on séquence le gène muté correspondant à ladite protéine mutée.

23. Procédé de mutagenèse d'une protéine cible ou d'une banque de protéines mutées cibles selon la revendication 21 ou de sélection d'une protéine mutée ou du gène correspondant selon la revendication 22, **caractérisé en ce qu'**il comprend la préparation d'une banque d'expression de gènes mutés à partir d'un gène cible codant pour ladite protéine selon l'une quelconque des revendications 16 à 20, **caractérisé en ce qu'**il comprend après l'étape (i), les étapes suivantes :
j) on incube le milieu sélectif à la température adéquate pendant le temps suffisant à la pousse de colonies bactériennes individuelles,
k) on ensemence des cultures de bactéries individuelles à partir dès colonies de l'étape (j),
l) on effectue des préparations d'ADN plasmidique des cultures de l'étape (k),
m) on mesure l'activité biologique associée à chaque préparation d'ADN plasmidique de l'étape (1) et on compare le résultat obtenu par rapport à celui mesuré en utilisant l'ADN plasmidique non muté.
n) éventuellement, on séquence les préparations d'ADN plasmidique ayant permis d'observer des modifications significatives de l'activité biologique.

## Claims

1. Method for the mutagenesis of a target gene, consisting in preparing a series of N oligonucleotides, each oligonucleotide having a sequence complementary to a different region of the target gene and from one to three mutations placed at the centre of the sequence of the oligonucleotide, then in reacting said series of oligonucleotides with said target gene under conditions that allow the production of copies of the target gene carrying at least one mutation, **characterized in that** the target gene is carried by a double-stranded circular plasmid, all N of said oligonucleotides of the series, with N greater than 5, covering all or part of the sequence of said target gene, and **in that** said series of oligonucleotides is reacted with the target gene in the presence of a polymerase so as to generate, after selection by means of a restriction enzyme that makes it possible to eliminate the DNA molecules methylated on the two strands and to conserve those that are not methylated or hemimethylated, a library of mutated genes in which each mutation originating from a different oligonucleotide is present, on average, in less than 1/5 of the genes of the library.

2. Method for the mutagenesis of a library of target mutated genes, consisting in preparing a series of N oligonucleotides, each oligonucleotide having a sequence complementary to a different region of the target mutated genes and from one to three mutations placed at the centre of the sequence of the oligonucleotide, then in reacting said series of oligonucleotides with said target mutated genes under conditions that allow the production of copies of the target mutated genes carrying at least one mutation, **characterized in that** the target mutated genes are carried by double-stranded circular plasmids, all N of said oligonucleotides of the series, with N greater than 5, covering all or part of the sequence of said target mutated genes, and **in that** said series of oligonucleotides is reacted with target mutated genes in the presence of a polymerase so as to generate, after selection by means of a restriction enzyme that makes it possible to eliminate the DNA molecules methylated on the two strands and to conserve those that are not methylated or hemimethylated, a library of mutated genes in which each mutation originating from a different oligonucleotide is present, on average, in less than 1/5 of the genes of the library.

3. Method of mutagenesis according to Claim 2, in which the library of target mutated genes has been obtained according to the method of Claim 1.

4. Method of mutagenesis according to Claims 1 to 3, **characterized in that** N is between 5 and 10⁶, and **in that** each mutation originating from a different oligonucleotide is present, on average, between 1/5 and 1/10⁶.

5. Method of mutagenesis according to one of Claims 1 to 4, **characterized in that** the series of oligonucleotides comprises N different oligonucleotides and **in that** each mutation originating from a different oligonucleotide is present in, on average, approximately 1/N of the genes of the library.

6. Method of mutagenesis according to any one of Claims 1 to 5, **characterized in that** the series of oligonucleotides is reacted with the target gene or the library of target mutated genes according to a molar ratio of each oligonucleotide relative to the target gene or to the library of target mutated genes of between 0.01 and 100.

7. Method of mutagenesis according to any one of Claims 1 to 6, **characterized in that** the series of oligonucleotides is reacted with the target gene or the library of target mutated genes in the presence of a polymerase that has a strand displacement activity, such as the Taq polymerase, or a 3' → 5' exonuclease activity, such as the Pfu polymerase.

8. Method of mutagenesis according to Claim 7,
**characterized in that** the series of oligonucleotides is reacted with the target gene or the library of target mutated genes in the presence of a polymerase that has a strand displacement activity or a 3' → 5' exonuclease activity and a ligase.

9. Method of mutagenesis according to any one of Claims 1 to 6, **characterized in that** the series of oligonucleotides is reacted with the target gene or the library of target mutated genes in the presence of a polymerase that has no strand displacement activity or 3' → 5' exonuclease activity, such as the T4 polymerase, and in the absence of ligase.

10. Method of mutagenesis according to any one of the preceding claims, **characterized in that** the oligonucleotides of the series may or may not be overlapping and have a size of between 10 and 100.

11. Method of mutagenesis according to any one of the preceding claims, **characterized in that** each oligonucleotide comprises from 1 to 3 mutation(s) placed at the centre of its sequence.

12. Method according to any one of the preceding claims, **characterized in that** the mutations of each oligonucleotide are chosen from deletions and/or insertions of one or more nucleotide(s).

13. Method of mutagenesis according to any one of the preceding claims, **characterized in that** each oligonucleotide of the series is present as several copies, each copy having a different nucleotide at the level of said mutation(s).

14. Method of mutagenesis according to any one of the preceding claims, **characterized in that** each mutation is of the type that makes it possible to introduce an identical codon into each oligonucleotide or a codon corresponding to the same amino acid, as a substitution for the original codon of the target gene.

15. Method of mutagenesis according to Claim 14, **characterized in that** said codon corresponds to an amino acid chosen from the group comprising Ala, Val, Gly, Leu and Ile.

16. Method of mutagenesis according to any one of Claims 1 to 15, **characterized in that** it comprises the following steps:
a) a template consisting of a plasmid containing the target gene or the library of target mutated genes and a resistance gene is prepared,
b) an equimolecular series of oligonucleotides having a sequence complementary to a different region of the target gene or the library of target mutated genes and at least one mutation placed at the centre of the sequence of the oligonucleotide is prepared, all the oligonucleotides of the series covering all or part of the sequence of said target gene or of said library of target mutated genes,
c) the series of oligonucleotides prepared in step b) is mixed with the plasmid of step (a) according to a molar ratio of each oligonucleotide relative to the plasmid of between 0.01 and 100,
d) the mixture of step (c) is temperature-denatured so as to obtain a single-stranded template,
e) the mixture of step (d) is subjected to a temperature that allows the oligonucleotides to hybridize on the template,
f) a polymerase, its buffer and its cofactors, and a sufficient amount of each of the nucleotide triphosphates, are added to the mixture so as to allow replication of the strands of the template from any of the oligonucleotides,
g) optionally, steps (d), (e) and (f) are repeated,
h) the products of step (f) or (g) that have undergone replication are selected by any appropriate means,
i) competent bacteria are transformed with the products selected in step (h), and the products carrying a mutated gene are selected on a selective medium corresponding to a resistance gene carried by the plasmid of step (a).

17. Method of mutagenesis according to Claim 16, **characterized in that** step (h) consists in subjecting the products of step (f) to the action of a restriction enzyme that selects the products having undergone replication.

18. Method of mutagenesis according to Claim 17, **characterized in that** the restriction enzyme that selects the products having undergone replication is the DpnI enzyme.

19. Method of mutagenesis according to any one of Claims 16 to 18, **characterized in that** the oligonucleotides are phosphorylated at their 5' end and **in that**, in step (f), a ligase is added.

20. Method of mutagenesis according to any one of the preceding claims, **characterized in that** the target gene is a nucleic acid molecule encoding a protein of interest with or without its own regulatory sequences.

21. Method for the mutagenesis of a target protein or of a library of target mutated proteins, **characterized in that** it comprises the preparation of an expression library of mutated genes based on a target gene encoding said protein, according to any one of Claims 1 to 20, then **in that** said mutated genes are expressed so as to produce a library of mutated proteins and, optionally, **in that** said mutated proteins are screened for a desired function.

22. Method for the selection of mutated proteins having a modified activity compared with the same non-mutated protein, or of the mutated gene corresponding to said mutated protein, **characterized in that** it comprises a method of mutagenesis according to Claim 21, and **in that**, after the screening of said mutated proteins for a desired function, the mutated protein exhibiting the desired function is selected and, optionally, the mutated gene corresponding to said mutated protein is sequenced.

23. Method for the mutagenesis of a target protein or of a library of target mutated proteins according to Claim 21 or for the selection of a mutated protein or of the corresponding gene according to Claim 22, **characterized in that** it comprises the preparation of an expression library of mutated genes based on a target gene encoding said protein, according to any one of Claims 16 to 20, and **characterized in that** it comprises, after step (i), the following steps:
j) the selective medium is incubated at the appropriate temperature for the period of time sufficient for the growth of individual bacterial colonies,
k) individual bacteria cultures are inoculated using the colonies of step (j),
l) plasmid DNA preparations are carried out from the cultures of step (k),
m) the biological activity associated with each plasmid DNA preparation of step (1) is measured and the result obtained is compared with that measured using the non-mutated plasmid DNA,
n) optionally, the plasmid DNA preparations having made it possible to observe significant modifications in the biological activity are sequenced.

## Patentansprüche

1. Verfahren zur Mutagenese eines Zielgens, das darin besteht, eine Reihe von N Oligonucleotiden herzustellen, wobei jedes Oligonucleotid eine Sequenz, die komplementär zu einem anderen Bereich des Zielgens ist, und ein bis drei Mutationen aufweist, die im Zentrum der Sequenz des Oligonucleotids angeordnet sind, anschließend die Reihe der Oligonucleotide mit dem Zielgen unter Bedingungen reagieren zu lassen, die die Erzeugung von Kopien des Zielgens ermöglichen, die mindestens eine Mutation aufweisen, **dadurch gekennzeichnet, dass** das Zielgen von einem doppelsträngigen ringförmigen Plasmid getragen wird, wobei die Gesamtheit N der Oligonucleotide der Reihe, mit N größer als 5, die gesamte Sequenz oder einen Teil der Sequenz des Zielgens abdeckt, und dass die Reihe der Oligonucleotide in Gegenwart einer Polymerase mit dem Zielgen umgesetzt wird, um nach Selektion mit einem Restriktionsenzym, das es ermöglicht, die DNA-Moleküle auszuschließen, die auf den beiden Strängen methyliert sind, und diejenigen zu behalten, die nicht methyliert oder halbmethyliert sind, eine Bank mutierter Gene zu erzeugen, worin jede Mutation, die von einem der verschiedenen Oligonucleotide stammt, im Mittel in weniger als 1/5 der Gene der Bank vorhanden ist.

2. Verfahren zur Mutagenese einer Bank mutierter Zielgene, das darin besteht, eine Reihe von N Oligonucleotiden herzustellen, wobei jedes Oligonucleotid eine Sequenz, die komplementär zu einem anderen Bereich der mutierten Zielgene ist, und ein bis drei Mutationen aufweist, die im Zentrum der Sequenz des Oligonucleotids angeordnet sind, anschließend die Reihe der Oligonucleotide unter Bedingungen mit den mutierten Zielgenen reagieren zu lassen, die die Erzeugung von Kopien der mutierten Zielgene ermöglichen, die mindestens eine Mutation aufweisen, **dadurch gekennzeichnet, dass** die mutierten Zielgene von doppelsträngigen ringförmigen Plasmiden getragen werden, wobei die Gesamtheit N der Oligonucleotide der Reihe, mit N größer als 5, die gesamte Sequenz oder einen Teil der Sequenz der mutierten Zielgene abdecken, und dass die Reihe der Oligonucleotide in Gegenwart einer Polymerase mit den mutierten Zielgenen umgesetzt wird, um nach Selektion mit einem Restriktionsenzym, das es ermöglicht, die DNA-Moleküle auszuschließen, die auf den beiden Strängen methyliert sind, und diejenigen zu behalten, die nicht methyliert oder hemimethyliert sind, eine Bank mutierter Gene zu erzeugen, worin jede Mutation, die von einem der verschiedenen Oligonucleotide stammt, im Mittel in weniger als 1/5 der Gene der Bank vorhanden ist.

3. Verfahren zur Mutagenese nach Anspruch 2, wobei die Bank der mutierten Zielgene nach dem Verfahren von Anspruch 1 erhalten wurde.

4. Verfahren zur Mutagenese nach den Ansprüchen 1 bis 3, **dadurch gekennzeichnet, dass** N im Bereich von 5 bis 10⁶ liegt und dass jede Mutation, die von einem der verschiedenen Oligonucleotide stammt, im Mittel in etwa 1/N bis 1/10⁶ vorhanden ist.

5. Verfahren zur Mutagenese nach einem der Ansprüche 1 bis 4, **dadurch gekennzeichnet, dass** die Reihe der Oligonucleotide N verschiedene Oligonucleotide umfasst und dass jede Mutation, die von einem der verschiedenen Oligonucleotide stammt, im Mittel in etwa 1/N der Gene der Bank vorhanden ist.

6. Verfahren zur Mutagenese nach einem der Ansprüche 1 bis 5, **dadurch gekennzeichnet, dass** die Reihe der Oligonucleotide mit dem Zielgen oder der Bank mutierter Zielgene in einem molaren Verhältnis jedes Oligonucleotides, bezogen auf das Zielgen oder die Bank mutierter Zielgene, umgesetzt wird, das im Bereich von 0,01 bis 100 liegt.

7. Verfahren zur Mutagenese nach einem der Ansprüche 1 bis 6, **dadurch gekennzeichnet, dass** die Reihe der Oligonucleotide mit dem Zielgen oder der Bank der mutierten Zielgene in Gegenwart einer Polymerase umgesetzt wird, die eine Strangverschiebungaktivität, wie der Taq-Polymerase, oder eine 3'→5'-Exonuclease-Aktivität hat, wie der Pfu-Polymerase.

8. Verfahren zur Mutagenese nach Anspruch 7, **dadurch gekennzeichnet, dass** die Reihe der Oligonucleotide mit dem Zielgen oder der Bank mutierter Zielgene in Gegenwart einer Polymerase, die eine Strangverschiebungsaktvität oder eine 3'→5'-Exonuclease-Aktivität hat, und einer Ligase umgesetzt wird.

9. Verfahren zur Mutagenese nach einem der Ansprüche 1 bis 6, **dadurch gekennzeichnet, dass** die Reihe der Oligonucleotide mit dem Zielgen oder der Bank mutierter Zielgene in Gegenwart einer Polymerase, die keine Strangverschiebungsaktvität oder 3'→5'-Exonuclease-Aktivität hat, wie der T4-Polymerase, und in Abwesenheit von Ligase umgesetzt wird.

10. Verfahren zur Mutagenese nach einem der vorhergehenden Ansprüche, **dadurch gekennzeichnet, dass** die Oligonucleotide der Reihe überlappend oder nicht überlappend sind und eine Größe aufweisen, die im Bereich von 10 bis 100 liegt.

11. Verfahren zur Mutagenese nach einem der vorhergehenden Ansprüche, **dadurch gekennzeichnet, dass** jedes Oligonucleotid 1 Mutation bis 3 Mutationen aufweist, die im Zentrum seiner Sequenz angeordnet ist/sind.

12. Verfahren nach einem der vorhergehenden Ansprüche, **dadurch gekennzeichnet, dass** die Mutationen jedes Oligonucleotids unter den Deletionen und oder den Insertionen eines oder mehrerer Nucleotide ausgewählt werden.

13. Verfahren zur Mutagenese nach einem der vorhergehenden Ansprüche, **dadurch gekennzeichnet, dass** jedes Oligonucleotid der Reihe in mehreren Exemplaren vorhanden ist, wobei jedes Exemplar ein anderes Nucleotid im Bereich der Mutation(en) aufweist.

14. Verfahren zur Mutagenese nach einem der vorhergehenden Ansprüche, **dadurch gekennzeichnet, dass** jede Mutation des Typs ist, der es ermöglicht, ein identisches Codon in jedes Oligonucleotid oder ein Codon, das der gleichen Aminosäure entspricht, einzuführen, unter Substitution des ursprünglichen Codons des Zielgens.

15. Verfahren zur Mutagenese nach Anspruch 14, **dadurch gekennzeichnet, dass** das Codon einer Aminosäure entspricht, die aus der Gruppe ausgewählt wird, die Ala, Val, Gly, Leu, Ile umfasst.

16. Verfahren zur Mutagenese nach einem der Ansprüche 1 bis 15, **dadurch gekennzeichnet, dass** es die folgenden Schritte umfasst:
a) Herstellen einer Matrix, die aus einem Plasmid besteht, das das Zielgen oder die Bank mutierter Zielgene und ein Resistenzgen enthält,
b) Herstellen einer äquimolekularen Reihe von Oligonucleotiden, die die komplementäre Sequenz voneinander verschiedener Bereiche des Zielgens oder der Bank mutierter Zielgene und mindestens eine Mutation aufweisen, die im Zentrum der Sequenz des Oligonucleotids angeordnet ist, wobei die Gesamtheit der Oligonucleotide der Reihe die gesamte Sequenz oder einen Teil der Sequenz des Zielgens oder der Bank der mutierten Zielgene abdeckt,
c) Vermischen der Reihe der Oligonucleotide, die in Schritt (b) hergestellt wurde, mit dem Plasmid aus Schritt (a) in einem molaren Verhältnis jedes Oligonucleotids, bezogen auf das Plasmid, das im Bereich von 0,01 bis 100 liegt,
d) Denaturieren des Gemischs aus Schritt (c) durch die Temperatur, um eine einzelsträngige Matrix zu erhalten,
e) Einwirken lassen einer Temperatur auf das Gemisch aus Schritt (d), die die Hybridisierung der Oligonucleotide auf die Matrix ermöglicht,
f) Zugeben einer Polymerase, ihres Puffers und ihrer Cofaktoren, und einer ausreichenden Menge jedes der Nucleotidtriphosphate zum Gemisch, um die Replikation der Stränge der Matrix mit beliebigen Oligonucleotiden zu ermöglichen,
g) gegebenenfalls Wiederholen der Schritte (d), (e) und (f),
h) Auswählen mit jedem geeigneten Mittel der Produkte aus Schritt (f) oder (g), die der Replikation unterzogen wurden,
i) Transformieren der in Schritt (h) ausgewählten Produkte in kompetente Bakterien, und Auswählen der Produkte, die ein mutiertes Gen tragen, auf einem selektiven Medium, das mit einem Resistenzgen korrespondiert, das von dem Plasmid aus Schritt (a) getragen wird.

17. Verfahren zur Mutagenese nach Anspruch 16, **dadurch gekennzeichnet, dass** der Schritt (h) darin besteht, die Produkte aus Schritt (f) der Einwirkung eines Restriktionsenzyms zu unterziehen, das die Produkte auswählt, die der Replikation unterzogen wurden.

18. Verfahren zur Mutagenese nach Anspruch 17, **dadurch gekennzeichnet, dass** das Restriktionsenzym, das die Produkte auswählt, die der Replikation unterzogen wurden, das Enzym DpnI ist.

19. Verfahren zur Mutagenese nach einem der Ansprüche 16 bis 18, **dadurch gekennzeichnet, dass** die Oligonucleotide an ihrem 5'-Ende phosphoryliert sind und dass im Schritt (f) eine Ligase zugegeben wird.

20. Verfahren zur Mutagenese nach einem der vorhergehenden Ansprüche, **dadurch gekennzeichnet, dass** das Zielgen ein Nucleinsäuremolekül ist, das ein interessierendes Protein mit oder ohne seine eigenen Regulationssequenzen codiert.

21. Verfahren zur Mutagenese eines Zielproteins oder einer Bank von mutierter Zielproteine, **dadurch gekennzeichnet, dass** es die Erzeugung einer Expressionsbank von mutierten Genen aus einem Zielgen umfasst, das das Protein nach einem der Ansprüche 1 bis 20 codiert, dass anschließend die mutierten Gene exprimiert werden, um eine Bank mutierter Proteine zu erzeugen, und dass die mutierten Proteine gegebenenfalls hinsichtlich einer gewünschten Funktion einem Screening unterzogen werden.

22. Verfahren zur Selektion mutierter Proteine, die eine modifizierte Aktivität aufweisen, bezogen auf das gleiche nicht mutierte Protein, oder des mutierten Gens, das dem mutierten Protein entspricht, **dadurch gekennzeichnet, dass** es ein Verfahren zur Mutagenese nach Anspruch 21 umfasst, dass nach dem Screening der mutierten Proteine hinsichtlich einer gewünschten Funktion das mutierte Protein ausgewählt wird, das die gewünschte Funktion aufweist.

23. Verfahren zur Mutagenese eines Zielproteins oder einer Bank mutierter Zielproteine nach Anspruch 21 oder zur Selektion eines mutierten Proteins oder des entsprechenden Gens nach Anspruch 22, **dadurch gekennzeichnet, dass** es die Erzeugung einer Expressionsbank mutierter Gene ausgehend von einem Zielgen umfasst, das das Protein nach einem der Ansprüche 16 bis 20 codiert, **dadurch gekennzeichnet, dass** es nach dem Schritt (i) die folgenden Schritte umfasst:
j) Inkubieren des selektiven Mediums bei der adäquaten Temperatur über den Zeitraum, der für das Wachstum individueller Bakterienkolonien ausreichend ist,
k) Beimpfen von Kulturen mit individuellen Bakterien aus den Kolonien aus Schritt (j),
l) Erzeugen von Zubereitungen plasmidischer DNA aus den Kulturen aus Schritt (k),
m) Messen der biologischen Aktivität, die jede Zubereitung plasmidischer DNA aus Schritt (1) aufweist, und Vergleichen des erhaltenen Ergebnisses, bezogen auf das Ergebnis, das bei Verwendung der nicht mutierten plasmidischen DNA erhalten wird,
n) gegebenenfalls Sequenzieren der Zubereitungen plasmidischer DNA, bei denen die Beobachtung signifikanter Änderungen der biologischen Aktivität möglich war.
